# EUROPEAN PATENT APPLICATION

(11) **EP 1 133 985 A1**
(43) Date of publication of application: **19.09.2001**
(21) Application number: 99972531.0
(22) Date of filing: 10.11.1999
(51) Int. Cl.: A61K 9/70, A61K 47/30, A61K 47/10, A61K 47/42, A61N 1/30

(54) **PRESSURE-SENSITIVE ADHESIVE GEL COMPOSITION FOR IONTOPHORESIS AND APPARATUS THEREFOR**

(30) Priority: 26.11.1998 JP 33560298
(71) Applicant: HISAMITSU PHARMACEUTICAL CO. INC., Tosu-shi, Saga-ken 841-0017 (JP)
(72) Inventor: KATAGAI, Kazuya, Tsukuba Laboratory, Tsukuba-shi, Ibaraki 305-0856 (JP); AADACHI, Hirotoshi, Tsukuba Laboratory, Tsukuba-shi, Ibaraki 305-0856 (JP); INOUE, Kazutaka, Tsukuba Laboratory, Tsukuba-shi, Ibaraki 305-0856 (JP)
(74) Representative: Westendorp, Michael Oliver
(86) International application number: JP9906241
(87) International publication number: WO0030621

(57) **Abstract**

The present invention provides an adhesive gel composition for an iontophoresis allowing a basic drug to be delivered efficiently into an in vivo site and a device therefore. This adhesive gel composition for an iontophoresis comprises (a) basic drug(s), an acidic polymer, a polyfunctional epoxy compound, water and a polyhydric alcohol and/or a gelatin, and the weight ratio of the basic drug(s) to the acidic polymer is preferably 10:1 to 1:10. The basic drug(s) is (are) preferably in a free form.

## Description

### TECHNICAL FIELD

The present invention relates an adhesive gel composition for an iontophoresis which is applied transdermally or transmucosally and which allows a basic drug to be delivered into an in vivo site utilizing an electrical driving force and a device therefore.

### BACKGROUND OF THE INVENTION

An iontophoresis is a transdermal and transmucosal absorption-enhancing system utilizing an electric driving force, and its principal is an enhancement of a skin barrier penetration of a drug molecule based on a force by which a positively-charged molecule is moved from a cathode to an anode and a negatively-charged molecule is moved from the anode to the cathode in an electric field generated between the cathode and the anode mainly by energization [See Journal of Controlled Release, Vol.18, 1992, pp.213 to 220; Advanced Drug Delivery Review, Vol.9, 1992, p.119; Pharmaceutical Research, Vol.3, 1986, pp.318 to 326].

As adhesive compositions which have been employed in a transdermal therapeutic system (TTS), many ones containing natural rubber-derived, synthetic rubber-derived, acrylic and silicone-derived lipophilic bases have been known. As a hydrophilic adhesive, a plaster containing a hydrophilic base such as a sodium polyacrylate and a carboxyvinyl polymer have been known. In addition, an adhesive composition obtained by crosslinking a methoxyethylene maleic anhydride copolymer with a polyfunctional epoxy compound for the purpose of increasing the adhesive performance, is disclosed in JP-A-56-154421 or JP-A-59-204117. As a patch for external use containing a basic drug, an adhesive gel containing lidocaine or its salt in a base having a water-soluble polymer, water and a moisturizing agent as essential components is disclosed in JP-A-4-305523.

On the other hand, a base used for administering a drug by means of an iontophoresis should be a water-soluble base. A karaya gum-derived base and a polyvinyl alcohol-derived base have been known as disclosed in JP-A-58-10050 or JP-A-63-92360. Such bases show problems of their low gel adhesive performances and poor compliances when used. Further, the adhesive performance of an adhesive composition having a metal-crosslinked water-soluble polymer as a base is reduced due to the reduction of the cohesive force of the gel upon energization. In order to dissolve the problems, an adhesive composition comprising a maleic anhydride copolymer or a maleic acid copolymer, a polyfunctional epoxy compound and a polyhydric alcohol is disclosed in JP-A-63-92683, and a base comprising a copolymer of acrylic acid and acrylamide is disclosed in JP-A-5-97662. However, these base compositions also involve additional problems such as a change in the physicochemical property or the stability over a time period and a reduction in the adhesive performance due to a reduced gel cohesion attributable to the addition of an electrolyte required in an iontophoresis. Such tendency becomes more evident especially when incorporating a basic drug or a salt.

Another adhesive gel composition whose impedance is low and whose salting-out and depolarization were improved, is disclosed in JP-A-9-286891 as a base for an iontophoresis. While this base composition for an iontophoresis can maintain a suitable adhesive performance and a low electric resistance even when a salt such as sodium chloride is incorporated, it has still problems of the insufficiency in the physicochemical property, the adhesive performance and the stability over a time period when a component causing a change in the pH of the gel such as an acidic agent or a basic drug is added.

The conventional lipophilic adhesive composition described above, however, had problems of requiring an organic solvent, much calories upon manufacturing and a surfactant as is experienced with an acrylic adhesive of an emulsion type. Such lipophilic adhesive composition Causes a relatively high skin irritation and has also problems in terms of the working environment in a manufacturing process. Furthermore, the plaster using such lipophilic adhesive had not been imparted with a sufficient adhesive performance.

The conventional hydrophilic adhesive compositions disclosed in JP-A-56-154421 and JP-A-59-204117 have also problems of a difficulty in controlling the thickness due to a low viscosity upon plastering. Moreover, in these bases, the physicochemical property of the gel varies significantly upon addition of an electrolyte such as a salt. A metal-crosslinked adhesive composition containing lidocaine as a basic drug is disclosed in JP-A-4-305523. However, in this adhesive composition using a Water-soluble polymer crosslinked with a metal as a base, the adhesive performance is reduced due to a reduction in the gel cohesion upon energization in an iontophoresis, and has problems of the safety because of the migration of metal ions into a skin especially in an iontophoresis from a cathode.

On the other hand, conventional karaya gum-derived base or polyvinyl alcohol-derived bases for an iontophoresis disclosed in JP-A-58-10050 and JP-A-63-92360, involve a less effect of an electrolyte on the gelation and can maintain a conductivity upon the energization. However, such base had problems of low adhesive performance, poor compliance by a user and poor stability over a time period.

A conventional hydrophilic adhesive composition disclosed in JP-A-63-92683 also involves a problem due to the fact that it should contain an increased amount of a base polymer for enabling a plastering because of its low viscosity upon plastering and that such base polymer should be neutralized with an alkali. Accordingly, with such conventional hydrophilic adhesive composition, an increase in the diffusion resistance of a drug or a reduction in the delivery rate is caused, resulting in a problem of a substantial reduction in the permeability of the drug.

While each of a base consisting of a copolymer of acrylic acid and acrylamide disclosed in JP-A-5-97662 and an adhesive composition consisting of a methoxymaleic anhydride copolymer or a methoxy maleic acid copolymer and an N-vinylacetamide crosslinked structure disclosed in JP-A-9-286891 undergoes no deterioration of the characteristics such as an adhesive performance upon addition of a neutral electrolyte or component, it allows the absorption to be reduced due to a reduction in the solubility of a drug in case of the addition of a component which causes the pH of the gel such as a basic drug. Also when an acidic pH modifier such as hydrochloric acid is added for suppressing the change in the pH of a gel, there is a problem that the moldability and the adhesive performance of the gel are reduced.

Accordingly, an object of the present invention is to solve the problems described above and to provide an adhesive gel composition for an iontophoresis enabling an efficient delivery of a basic drug into an in vivo site as well as a device therefore.

### DISCLOSURE OF THE INVENTION

We made an effort to accomplish the object described above and finally established the invention. The adhesive gel for an iontophoresis according to the present invention comprises (a) basic drug(s), an acidic polymer, a polyfunctional epoxy compound, water, a polyhydric alcohol and/or a gelatin. In this composition, the weight ratio of the basic drug(s) to the acidic polymer is preferably 10:1 to 1:10.

The acidic polymer is one or more selected from the group consisting of a polyacrylic acid, a methoxyethylene maleic anhydride copolymer, a methoxyethylene maleic acid copolymer, an isobutylene maleic anhydride copolymer, an isobutylene maleic acid copolymer, a carboxyvinyl polymer and carboxymethyl cellulose.

The basic drug is preferably in a free form. The basic drug may for example be a local anesthetic agent, preferably a combination of a local anesthetic agent and a vasoconstrictor. As the local anesthetic agent, lidocaine is used and as the vasoconstrictor, epinephrine is used. In such case, the weight ratio of lidocaine to epinephrine is preferably 1000:1 to 2:1. Lidocaine is preferably contained in an amount of 1 to 20 % by weight, and epinephrine is preferably contained in an amount of 0.001 to 0.5 % by weight. It is preferable that an antioxidant is further contained. The antioxidant is one or more selected from the group consisting of sodium pyrosulfite, sodium hydrogen sulfite and oxyquinoline sulfate. The antioxidant is preferably contained in an amount of 0.001 to 1.0 % by weight.

An iontophoresis device according to the present invention comprising a donor electrode, a reference electrode and a power supply connected electrically to each of the donor electrode and the reference electrode, wherein the current output from the power supply device is at least one of a direct current, a pulse direct current and a pulse depolarized direct current and wherein the current rate is 0.5 to 2.0 mA·min/cm².

By the structure described above, an adhesive gel composition of a matrix type for an iontophoresis and a device therefore can be obtained to deliver a basic drug efficiently. With the adhesion gel composition and the device therefore, reduction in the drug derivery is not caused, and moldability and adhesive performance of a gel are excellent, and has a low skin irritation, together with a further advantage of a capability of being produced readily and at a low cost by means of plastering or filling.

### BRIEF DESCRIPTION OF THE DRAWINGS

Figure 1 shows a schematic sectional view of an applicator in the iontophoresis device according to the present invention.
Figure 2 shows a schematic sectional view of one embodiment of the iontophoresis device according to the invention.

### BEST MODE FOR CARRYING OUT THE INVENTION

A preferred embodiment of an adhesive composition for an iontophoresis containing a basic drug and a device therefor according to the invention will be detailed below.

An adhesive gel composition of the invention comprises an acidic polymer, a polyfunctional epoxy compound, water, a polyhydric alcohol and/or a gelatin and a basic drug, and by adjusting the weight ratio of the acidic polymer to the basic drug, an iontophoresis composition having excellent moldability and adhesive performance can be obtained without reducing the drug delivery rate.

For example, an acidic polymer in an adhesive gel composition is preferably a water-soluble polymer, and for example, by employing one or more selected from the group consisting of a polyacrylic acid, a methoxyethylene maleic anhydride copolymer, a methoxyethylene maleic acid copolymer, an isobutylene maleic anhydride copolymer, an isobutylene maleic acid copolymer, a carboxyvinyl polymer and carboxymethyl cellulose, as a base polymer, it becomes possible to obtain an ability to adhere to a skin or a mucosa, and also by employing as a crosslinking agent, a polyfunctional epoxy compound, it becomes possible to suppress a reduction in the cohesive force of the composition. Furthermore, by adding a free form of a basic drug to neutralize the carboxyl group of a base polymer, it becomes possible to suppress a reduction in the cohesive force without the need of any additives such as a pH modifier, to increase the pH, to reduce the skin irritating effect and to reduce the competition between the drug and a hydrogen ion upon energization of an iontophoresis, whereby increasing the permeability of the drug. Furthermore, the addition of a polyhydric alcohol increases the cohesive force, the water-retaining ability and the drug solubility of an adhesive composition, and the addition of a gelatin improves the moldability of a gel, whereby simplifying the plastering performance and the cutting during a manufacturing process.

As described above, a combination of a basic drug and an acidic polymer according to the present invention allows the pH of a base to be adjusted within a weakly acidic to neutral range and provides suitable gel moldability and adhesive performance. On the other hand, a base composition consisting of a combination of a basic drug and a basic polymer or a neutral polymer allows the pH of a gel to shift substantially to a basic pH, which leads to a reduction not only in the solubility of a drug but also in the dissociation of an ion, resulting in a reduction also in the absorbability. A further addition of an acidic pH modifier such as hydrochloric acid, reduces the gel forming ability and the adhesive performance. There is a problem that a base composition containing a neutral polymer such as a polyvinyl alcohol and a polyvinylpyrrolidone as a base polymer suffers from an insufficient adhesive performance which leads to a problematically poor compliance when used.

While the weight ratio of (a) basic drug(s) to an acidic polymer in the present invention is not particularly limited as long as it is of a combination capable of adjusting the pH of a base within a weakly acidic to neutral region, it is preferably 10:1 to 1:10 by which the moldability and the adhesive performance of a gel and the diffusion and the release of the drug are not affected adversely. More preferably, the ratio is 5:1 to 1:5, particularly 3:1 to 1:3. Within the range specified above, the gel can be adjusted to a weakly acidic to neutral pH, e.g., pH 3 to 7. To adjust the pH within the range specified above is preferable also in view of the handling, the practicality and the viscosity upon manufacturing, since the skin irritating effect is low and a suitable adhesion performance can be obtained.

While an acidic polymer according to the present invention is not particularly limited, as long as it is a material which is a hydrophilic adhesive base capable of being produced without using any solvent and which has a low skin irritating effect and a suitable adhesion performance, an acidic polymer may, for example, be a polyacrylic acid, a methoxyethylene maleic anhydride copolymer, a methoxyethylene maleic acid copolymer, an isobutylene maleic anhydride copolymer, an isobutylene maleic acid copolymer, a carboxyvinyl polymer or carboxymethyl cellulose. By employing such acidic polymer as a base polymer, a reduction in the delivery rate due to a competitive ion, i.e., a reduction in the permeability of a drug can be suppressed. The amount of an acidic polymer contained in the composition of the present invention is preferably 1 to 20 % by weight, more preferably 1 to 10 % by weight. An amount less than 1 % by weight tends to result in a difficulty in obtaining a gel-forming ability and also to give a reduced adhesive performance, while an amount exceeding 20 % by weight leads to a potent cohesive force, which is not desirable in view of the manufacturing processes such as plastering and filling.

Further, a polymer which may be added for the purpose other than imparting an adhesive performance, such as a tackifier and a binder, includes a polysaccharide such as agar, starch, mannan, xanthane gum, locust bean gum, carrageenan, gellan gum, tamarind gum, curdlan, pectin, furcelleran, guar gum, alginic acid, sodium alginate, tara gum, karaya gum, gum arabic, cellulose or its derivative, a sodium polyacrylate, a polyvinyl alcohol, a polyvinyl pyrrolidone and the like, which may be used alone or in combination.

A polyfunctional epoxy compound used in the invention may, for example, be sorbitol polyglycidylether, polyglycerol polyglycidylether, diglycerol polyglycidylether, glycerol polyglycidylether, ethylene glycol diglycidylether, polyethylene glycol diglycidylether, propylene glycol diglycidylether, polypropylene glycol diglycidylether and the like. The amount of a polyfunctional epoxy compound contained in the composition according to the invention is preferably 0.01 to 2 % by weight based on the entire base. An amount less than 0.01 % by weight tends to give a difficulty in obtaining the moldability of a gel, while an amount exceeding 2 % by weight tends to allow an adhesive performance to be lost.

A polyhydric alcohol used in the present invention may, for example, be ethylene glycol, diethylene glycol, polyethylene glycol, propylene glycol, dipropylene glycol, polypropylene glycol, 1,3-butylene glycol, glycerin and a sugar alcohol such as D-sorbitol and D-mannitol, with polyethylene glycol and glycerin being preferred. Any of those listed above can be used alone or in combination. The amount of a polyhydric alcohol contained in the composition of the present invention is preferably 10 to 60 % by weight. An amount less than 10 % by weight tends to give a difficulty in obtaining a sufficient cohesive force, while an amount exceeding 60 % by weight tends to allow an adhesive performance to be lost.

Water used in the invention is essential for swelling a corneal layer of a skin, improving the permeability of a drug and obtaining an electric conductivity, and the amount of water to be contained is preferably 10 to 80 % by weight, more preferably 20 to 50 % by weight.

A gelatin contained in an adhesive composition according to the invention may be any one which was treated with an acid or an alkali, and the amount of the gelatin contained is preferably 0.1 to 15 % by weight. An amount less than 0.1 % by weight tends to give a difficulty in obtaining a sufficient cohesive force, while an amount exceeding 15 % by weight tends to give a difficulty in obtaining a suitable adhesive performance. If necessary, other additives such as an electrolyte, a pH modifier, a stabilizer, a tackifier, a humectant, a surfactant, a solubilizing agent and an absorption enhancer may also be added, as long as the moldability and the adhesive performance of a gel and the absorption performance of an agent are not affected adversely. For example, an electrolyte may for example be sodium chloride, potassium chloride, calcium chloride, magnesium chloride, an cation exchange resin, an anion exchange resin and the like. The amount of an electrolyte contained in the composition according to the invention is not particularly limited.

A basic drug(s) employed in the invention is(are) not particularly limited and may be any of those having pharmaceutical activities, such as antibiotics, antifungal agents, cardiotonics, antiarrhythmic agents, vasodilators, diuretics, hypotensive agents, hypolipidemic agents, circulatory agents, antiplatelet agents, hemostatic agents, anticoagulants, anti-tumor agents, antipyretics, analgesics, antiinflammatory agents, expectorants, sedatives, muscle relaxants, antiepileptics, antidepressants, anti-ulcer agents, antiallergics, antidiabetic agents, anti-tuberculosis agents, hormones, anti-narcotic agents, bone resorption suppressants, anti-angiogenetic agents, local anesthetics and the like.

While either of a free form or a salt of a basic drug may be used as long as the moldability and the adhesive performance of a gel are not affected adversely, a free form is preferred.

An antibiotic may, for example, be gentamycin, lividomycin, sisomicin, tetracycline, ampicillin, cefotiam, cefazolin, thienamycin, sulfazecin, streptomycin, kanamycin and rifampicin.

An antifungal agent may, for example, be amphotericin B, itraconazole, fluconazole and 2-[(1R, 2R)-2-(2,4-difluorophenyl-2-bydroxy-1-methyl-3-(1H-1,2,4-triazol-1-yl)propyl]-4-[4-2,2,3,3-tetrafluoropropoxy]phenyl)-3(2H,4H)-1,2,4-triazolone.

A cardiotonic may, for example, be dopamine, dobutamine and ubidecarenone. An antiarrhythmic agent may, for example, be procaineamide, lidocaine, phenytoin, propranol, metroprolol, verapamil, diltiazem and oxypurinol. A vasodilator may, for example, be nicardipine, verapamil, papaverine and tolazoline. A diuretic may, for example, be acetazolamide, methazolamide, chlorothiazide, furosemide, triamterene, amiloride and aminometradine. A hypotensive agent may, for example, be hydralazine, budralazine, prazosin, doxazocin, carteolol, clonidine, enalapril, captopril, delapril, manidipine, pinacidil and minoxidil. A circulatory agent may, for example, be etilefrin and dihydroergotamine. An antiplatelet agent may, for example, be ticlopidine. A hemostatic agent may, for example, be epinephrine.

An anti-tumor agent may for example be 6-mercaptopurine, cytarabine, bleomycin, actinomycin D, mitomycin C, adriamycin, procarbazine and doxorubicin.

An antipyretics, analgesic or-antiinflammatory agent may, for example, be tiaramide, emorfazone, buoprenorphine, eptazocine, pentazocine, butorphanol, tramazole, meperidine, morphine, hydromorphine and phentanyl. A muscle relaxant may, for example, be eperisone, tizanidine, chlorphenesin and tolperisone. An antidepressant may, for example, be amitriptyline, imipramine, clomipramine, desipramine and maprotiline.

An expetrorant may, for example, ambroxol, bromhexine, ephedrine, salbutamol, tulobuterol, formoterol, azelastin, ketotifen, codeine and picoperidamine.

A sedative may, for example, be chlorpromazine, fluphenazine and atropin. A muscle relaxant may, for example, be dantrolene, eperisone, tizanidine, tolperisone, pridinol and tubocurarine. An antiepileptic may, for example, be phenytoin and ethosuximide. An antidepressant may, for example, be imipramine and phenelzine.

An anti-ulcer agent may, for example, be cimetidine and famotidine. An antiallergic may, for example, be diphenylhydramine, chlorphenylamine, cyproheptazine, ketotifen, epinastine, tripelennamine and clemizole.

An antidiabetic agent may, for example, be tolbutamide, chlorpropamide, glibenclamid, acetohexamide, midaglizole, glymidine, glipizide and metformin.

An anti-tuberculosis agent may, for example, be streptomycin, kanamycin, isoniazid, ethambutol and pyrazinamide.

An anti-narcotic agent may, for example, be protamine, naloxone, revallorphan and nalorphine. A bone resorption suppressant may for example be raloxifene and alendronate.

A local anesthetic may, for example, be lidocaine, tetracaine, procaine, benzocaine, etidocaine, prilocaine, dibucaine, bupivacaine, proparacaine, phenacaine, cocaine, oxyprocaine, propitocaine, ethyl aminobenzoate, orthocaine, oxethazaine, mepivacaine and chloroprocaine.

A basic drug used in the present invention may, for example, be a local anesthetic listed above, and a more preferred basic drug is a combination of a local anesthetic and a vasoconstrictor. A representative local anesthetic is 5 lidocaine, whose surface infiltration and transmission anesthetic effect is employed in a nerve blocking therapy against a herpes zoster pain and a post-herpes zoster pain and also in a pain relief during a continuous intravenous puncture. In an iontophoresis composition comprising an acidic polymer and (a) basic drug(s) in the invention, the local anesthetic effect of lidocaine can be modified to a shorter-acting effect, an increased effect and a prolonged effect by combining with a vasoconstrictor epinephrine. This is attributable to an efficient absorption of a drug due to less competitive ion species coexisting in a base in a base composition of the invention.

In an iontophoresis composition for a local anesthesia according to the invention, the weight ratio of an acidic polymer which is one or more selected from the group consisting of a polyacrylic acid, a methoxyethylene maleic anhydride copolymer, a methoxyethylene maleic acid copolymer, an isobutylene maleic anhydride copolymer, an isobutylene maleic acid copolymer, a carboxyvinyl polymer and carboxymethyl cellulose and a basic drug comprising lidocaine and epinephrine, is not limited as long as it provides a combination allowing the pH of a base to be adjusted within a weakly acidic to neutral range, and is preferably 10:1 to 1:10 by which the moldability and the adhesive performance of a gel and the diffusion and the release of the agent are not affected adversely. More preferably, the ratio is 5:1 to 1:5, most preferably 3:1 to 1:3. In addition, while the weight ratio of lidocaine to epinephrine as basic drugs is not limited particularly, it is preferably 1000:1 to 2:1. A gel prepared within the range specified above has a pH which is adjusted within a weakly acidic to neutral range of pH 3 to 7. To adjust the pH and the adhesive performance as specified above is preferable also in view of the handling, the practicality and the viscosity upon manufacturing, since the skin irritating effect is reduced and a suitable adhesion performance can be obtained.

The amount of lidocaine contained in the composition according to the present invention is 1 to 20 % by weight, more preferably 1 to 10 % by weight. The amount of epinephrine contained is 0.001 to 0.5 % by weight, more preferably 0.01 to 0.5 % by weight. An amount of lidocaine less than 1 % by weight results in an insufficient absorption performance, while an amount exceeding 20 % by weight results in a high cohesive force, which is less desirable in manufacturing processes such as plastering. An amount of epinephrine less than 0.001 % by weight tends to give a difficulty in increasing the effect of lidocaine, while an amount exceeding 0.5 % by weight is results in a threshold of the pharmacological effect.

While an antioxidant used in the composition according to the invention is added for the purpose of preventing the oxidation of epinephrine, and its type is not particularly limited, a water-soluble antioxidant is used preferably, and a material having less competitive ion species is preferred. For example, one or more selected from the group consisting of L-ascorbic acid, erythorbic acid, citric acid, sodium edetate, sodium pyrosulfite, sodium hydrogen sulfite and oxyquinoline sulfate are used. In particular, sodium hydrogen sulfite and oxyquinoline sulfate are useful since they can be added without impairing the physicochemical property of an adhesive gel because of their antioxidative effects at low doses. The amount of sodium hydrogen sulfite or oxyquinoline sulfate to be added is 0.001 % by weight to 1.0 % by weight, more preferably 0.001 % by weight to 0.5 % by weight, especially 0.001 to 0.3 % by weight.

A composition of a reference electrode as a constituent of an iontophoresis device described above comprises, similarly to an adhesive gel composition of a donor electrode containing an agent described above, a base component comprising an acidic polymer, one or two of a polyfunctional epoxy compound or a metal compound, water, a polyhydric alcohol and/or a gelatin together with an electrolyte for improving the conductivity. A crosslinking agent used in a reference electrode in the invention is not particularly limited, and may be one or more of a polyfunctional epoxy compound or a metal compound, examples of the latter being aluminum hydroxide, aluminum silicate, magnesium hydroxide and calcium hydroxide. An electrolyte may, for example, be chlorides, phosphates or sulfates of sodium, potassium, calcium and magnesium, cation exchange resins and anion exchange resins. As an electrolyte, a pH modifier may be used. Such pH modifier may, for example, be amines such as triethanolamine and diethanolamine, sodium hydroxide, salts of acidic polymers such as sodium polyacrylate and sodium carboxymethyl cellulose as well as a basic polymer. A desirable physicochemical property of such reference electrode involves a viscosity, a moldability and an adhesive performance which are similar to those of a donor electrode. Thus , it is preferred that the gel of a reference electrode is adjusted at a weakly acidic to neutral pH of 3 to 7 and its adhesive performance is at a similar degree. By exhibiting an adhesive performance at a degree similar to that of a donor electrode, a less skin irritation and a suitable adhesive performance can be achieved. In addition, with regard to the binding performance upon application of a donor electrode, a reference electrode and a power supply connected thereto onto a skin, no discomfort in the application is experienced, thus providing a well-balanced formulation. Also since the base compositions of a donor electrode and a reference electrode are similar to each other, manufacturing processes such as plastering and filling are useful.

While an iontophoresis device according to the invention is not limited particularly with regard to the numbers of donor electrodes and reference electrodes, it comprises at least one pair of a donor electrode, a reference electrode and one power supply connected electrically to the donor electrode and the reference electrode. Each of the donor electrode and the reference electrode, is laminated with a respective adhesive gel. An electrode employed in the invention is not particularly limited as long as it is formed from a conductive electrode material which can usually be employed in an iontophoresis. Such material may, for example, be silver, silver chloride, aluminum, zinc, copper, iron, carbon, platinum, titanium and stainless steel. Among these, silver and silver chloride are satisfactory in terms of the electric characteristics such as a resistance, and are less expensive when used as pastes and give high producibilities.

A device of the invention is useful in administering an agent transdermally by means of an iontophoresis utilizing various applicators capable of being applied to a skin.

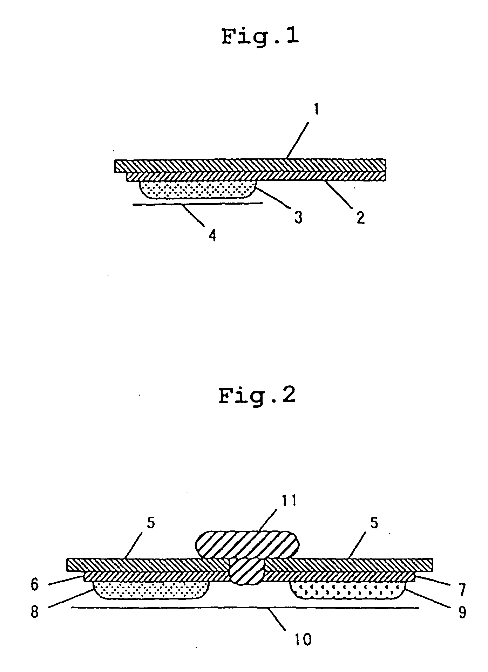

Figure 1 shows a schematic sectional view of an applicator fitted with an adhesive gel composition according to the invention. As shown in Figure 1, a basic part of the applicator comprises a backing (made from polyethylene terephthalate) 1 over which an electrode (silver paste) 2 is laminated, an adhesive gel 3 placed on the electrode 2 and a liner (made from polyethylene terephthalate) 4 covering the adhesive gel 3. The electrode 2 has an introduced electrode 5 print which enables a connection to an electrode directly or via a connector. The device thus formed is used by removing the liner 4 immediately before use followed by bringing the surface of the adhesive gel 3 into a direct contact with a skin.

The factors for reinforcing this device, i.e., the composition of a backing, the structures of a donor electrode and a reference electrode, the design as an integrated structure on an identical support or as a separated structure on several supports, are not limited particularly. In addition, the numbers of the constituents of the donor electrode and the reference electrode are not limited, and each may be at least one. The form of such device may include, but not limited to, rectangular, circular, oval, square, heart-shaped and diamond-shaped forms. The size and the color may not be limited as long as they are practical.

Figure 2 shows a schematic sectional view of an iontophoresis device fitted with an adhesive gel composition according to the invention. As shown in Figure 2, this device comprises a backing 5 on which a donor electrode 6 and a reference 7 are laminated, a donor electrode-side composition 8 placed on the donor electrode 6, a reference electrode-side composition 9 placed on the reference electrode 7, a liner 10 covering the both compositions 7 and 8 and a power supply 11. One pole of the power supply 11 is connected to the donor electrode 6, and the other pole is connected to the electrode 7. This device is used by removing the liner 10 and applying the surfaces of the donor electrode-side composition 6 and the reference electrode-side composition 7 directly onto a skin.

An iontophoresis device according to the invention can be performed, for example when it is used for a local anesthesia, by using at least one of a direct current, a pulse direct current and a pulse depolarized direct current and energizing by applying the direct voltage between the electrodes of the device. A power supply is preferably one capable of applying a continuous direct-current voltage and a pulse direct-current voltage. The frequency of the pulse direct-current voltage is preferably 0.1 to 200 kHz, more preferably 1 to 100 kHz, especially 5 to 80 kHz. The ON/OFF ratio of the pulse direct-current voltage is selected suitably within the range of 1/100 to 20/1, preferably 1/50 to 15/1, more preferably 1/30 to 10/1.

In such case, the time period during which the electricity is supplied is not limited particularly, and may be 60 minutes or less, more preferably 30 minutes or less, particularly 15 minutes or less. The total amount of the current is 0.25 to 5.00 mA·min/cm², more preferably, 0.5 to 2.0 mA·min/cm². The total amount of the current referred here means the product of the current and the time consumed for the permeation of an agent, and the current when applying a pulse direct current or a pulse depolarized direct current means a permeated current.

### (Examples)

The invention will be further described in detail referring to Examples and Comparative Examples based on Experiments, which are not intended to restrict the invention. In the following description, a % means a % by weight unless otherwise specified.

In Experiments, the physical characteristics of a gel were evaluated according to the criteria shown in Table 1 on the basis of the moldability, the skin touch and the adhesive performance. The pH of the surface of a gel was determined by a surface pH measuring electrode (HORIBA, Model 6261-10C Electrode). Indexes of the moldability were an elasticity value (SUN KAGAKU, Rheometer Model CR-200D) and a skin touch score (exudation), and an index of the adhesive performance was a probe tack value (RIGAKUKOGYO, Probe tack tester).

**Table 1**

| Moldability (elasticity level: g), | | Skin touch (exudation) | Adhesive performance (Tack value: gF) | | Evaluation |
|---|---|---|---|---|---|
| 0-50 | (Low) | Observed | Less than 1 | (Low) | × |
| 50-100 | (Moderate) | Almost not observed | 1-5 | (Moderate) | Δ |
| 100- | (High) | Not observed | 5 or more | (High) | O |

Further, the pharmacological effect of a preparation containing a local anesthetic agent was also evaluated. Before initiating the experiment, the dorsal region of a guinea pig was clipped using a clipper and an electric shaver, and the skin surface was wiped thoroughly with a gauze soaked with a slightly warm water. The right or left side of the dorsal medium line was stimulated with a stimulating needle, and a site where a skin constriction response was surely identified was employed as an adhesive gel-application site, and the remaining clipped region was applied with a control electrode. A drug-containing adhesive gel (donor electrode, 6 cm²) was connected to a cathode, and a sodium chloride-containing electrode (reference electrode, 6 cm²) was connected to an anode, whereby initiating the energization. The supply of electricity was performed with a direct current of 0.1 mA/cm² for 15 minutes. After completing the energization, the donor electrode-applied site was stimulated 6 times with the stimulating needle and the change in the skin constriction response was observed at intervals. The local anesthetic effect was judged with the scale of - to ++ shown in Table 2 with regard to the anesthetic effect immediately after the energization, while the anesthetic effect-sustaining effect was designated as O when the effect was sustained for 30 minutes or longer, as Δ when the effect was sustained for a period of 15 minutes or longer but shorter than 30 minutes, and as × when almost no effect was observed.

**Table 2**

| Response frequency | Anesthesia score immediately after energization |
|---|---|
| Responded to 5 to 6 times | - |
| Responded to 3 to 4 times | (±) - ± |
| Responded to 1 to 2 times | + |
| No response | ++ |

### <Experiment 1>

Table 3 shows exemplified composition in which a basic drug and an acidic polymer or a neutral polymer are used in combination according to the invention. By employing lidocaine or lidocaine hydrochloride as a basic drug, a polyacrylic acid as an acidic polymer, and a polyvinyl alcohol as a neutral polymer, the adhesive compositions of Examples 1, 2 and Comparative Examples 1, 2 were prepared.

**Table 3**

| Basic drug | Acidic polymer, Polyacrylic acid | Neutral polymer, Polyvinyl alcohol |
|---|---|---|
| Lidocaine | Example 1 | Comparative Example 1 |
| Lidocaine hydrochloride | Example 2 | Comparative Example 2 |

### (Example 1)

| | |
|---|---|
| Polyacrylic acid | 8 Parts by weight |
| Glycerin | 40 Parts by weight |
| Ethylene glycol diglycidylether | 0.1 Part by weight |
| Water | 43.9 Parts by weight |
| Lidocaine | 8 Parts by weight |

These components in the above formulation were mixed and stirred with heating at 50°C until a uniform mixture was obtained and a crosslinking agent was added finally at 40°C to obtain an adhesive composition of the present invention. The composition was plastered onto a release-treated PET liner to have a thickness of 1 mm, and cut into a suitable form and subjected to a test.

### (Example 2)

| | |
|---|---|
| Polyacrylic acid | 8 Parts by weight |
| Glycerin | 40 Parts by weight |
| Ethylene glycol diglycidylether | 0.1 Part by weight |
| Water | 43.9 Parts by weight |
| Lidocaine hydrochloride | 8 Parts by weight |

These components in the above formulation were mixed and stirred with heating at 50°C until a uniform mixture was obtained and a crosslinking agent was added finally at 40°C to obtain an adhesive composition of the present invention. The composition was plastered onto a release-treated PET liner to have a thickness of 1 mm, and cut into a suitable form and subjected to a test.

### (Comparative Example 1)

| | |
|---|---|
| Polyvinyl alcohol | 12 Parts by weight |
| Water | 80 Parts by weight |
| Lidocaine | 8 Parts by weight |

These components in the above formulation were stirred and dissolved with heating until a uniform mixture was obtained, and the solution was plastered onto a release-treated PET liner to have a thickness of 1 mm, and frozen at -80°C to effect crosslinking, so as to obtain a comparative adhesive composition. The resultant adhesive composition was cut into a suitable form and subjected to a test.

### (Comparative Example 2)

| | |
|---|---|
| Polyvinyl alcohol | 12 Parts by weight |
| Water | 80 Parts by weight |
| Lidocaine hydrochloride | 8 Parts by weight |

These components in the above formulation were stirred and dissolved with heating until a uniform mixture was obtained, and the solution was plastered onto a release-treated PET liner to have a thickness of 1 mm and frozen at -80°C to effect crosslinking, so as to obtain a comparative adhesive composition.
The resultant adhesive composition was cut into a suitable form and subjected to a test.

The pH, the moldability, the skin touch and the adhesive performance of the adhesive compositions of Examples 1, 2 and Comparative Examples 1 and 2 were evaluated. As a result, each of the combinations of the basis agents and the acidic polymers of Examples 1 and 2 exhibited excellent physical properties with regard to the moldability and the adhesive performance of the gel. Also since the pH of the gel was 5 or lower to allow the basic drug to be presented as being dissociated, that the presence as an ion which is advantageous in an iontophoresis was suggested. On the other hand, each of Comparative Examples 1 and 2 had a high gel pH indicating that the solubility of 5 the agent was reduced and the possibility of the presence as an ion was reduced, and its gel exhibited an extremely low adhesive performance.

**Table 4**

| | PH | Moldability | Skin touch | Adhesive performance |
|---|---|---|---|---|
| Example 1 | 4.8 | O | Δ | O |
| Example 2 | 3.8 | Δ | Δ | O |
| Comparative Example 1 | 8.2 | O | Δ | × |
| Comparative Example 2 | 6.6 | O | Δ | x |

### <Experiment 2>

Table 5 shows exemplified composition in which a basic drug and an acidic polymer were used in combination using a neutralizing agent. The adhesive compositions of Comparative Examples 3 to 5 using lidocaine as a basic drug which were added 5 by sodium hydroxide as a neutralizing agent, were prepared.

**Table 5**

| Basic drug | Without neutralizing agent | With neutralizing agent | With neutralizing agent | With neutralizing agent |
|---|---|---|---|---|
| Lidocaine | Example 1 | - | - | - |
| Lidocaine hydrochloride | Example 2 | Comparative Example 3 | Comparative Example 4 | Comparative Example 5 |

### (Comparative Example 3)

| | |
|---|---|
| Polyacrylic acid | 8 Parts by weight |
| Glycerin | 40 Parts by weight |
| Ethylene glycol diglycidylether | 0.1 Part by weight |
| Water | 42.9 Parts by weight |
| Sodium hydroxide | 1.0 Part by weight |
| Lidocaine hydrochloride | 8 Parts by weight |

These components in the above formulation were mixed and stirred with heating at 50°C until a uniform mixture was obtained, and a crosslinking agent was added finally at 40°C to obtain an adhesive composition of the present invention. The composition was plastered onto a release-treated PET liner to have a thickness of 1 mm ,and cut into a suitable form and subjected to a test.

### (Comparative Example 4)

| | |
|---|---|
| Polyacrylic acid | 8 Parts by weight |
| Glycerin | 40 Parts by weight |
| Ethylene glycol diglycidylether | 0.1 Part by weight |
| Water | 41.9 Parts by weight |
| Sodium hydroxide | 2.0 Parts by weight |
| Lidocaine hydrochloride | 8 Parts by weight |

These components in the above formulation were mixed and stirred with heating at 50°C until a uniform mixture was obtained, and a crosslinking agent was added finally at 40°C to obtain an adhesive composition of the present invention. The composition was plastered onto a release-treated PET liner to have a thickness of 1 mm, and cut into a suitable form and subjected to a test.

### (Comparative Example 5)

| | |
|---|---|
| Polyacrylic acid | 8 Parts by weight |
| Glycerin | 40 Parts by weight |
| Ethylene glycol diglycidylether | 0.1 Part by weight |
| Water | 39.9 Parts by weight |
| Sodium hydroxide | 4.0 Parts by weight |
| Lidocaine hydrochloride | 8 Parts by weight |

These components in the above formulation were mixed and stirred with heating at 50°C until a uniform mixture was obtained and a crosslinking agent was added finally at 40°C to obtain an adhesive composition of the present invention. The composition was plastered onto a release-treated PET liner to have a thickness of 1 mm, and cut into a suitable form and subjected to a test.

As shown in Table 6, the combination of the polyacrylic acid as an acidic polymer and lidocaine as a basic drug as in Example 1 exhibited an excellent characteristics in both of the moldability and the adhesive performance, while the composition of Example 2 had a low pH and a slightly poor moldability. Accordingly, sodium hydroxide as a pH modifier was added to obtain the adhesive compositions of Comparative Examples 3 to 5. As a result, the gel moldability and the skin touch were improved by an elevated pH, but the adhesive performance tended to be affected adversely.

The adhesive compositions of Examples 1, 2 and Comparative Examples 3 to 5 were employed also for evaluating the local anesthetic effect by means of a pin pricking method in guinea pigs. As a result, each of the adhesive compositions of Comparative Examples 4 and 5 exhibit reduced pharmacological effects when compared with Example 1 as shown in Table 6. This was considered to be attributable to a reduction in the local anesthetic effect due to the competition between the drug and sodium ion derived from sodium hydroxide as a pH modifier added for the purpose of improving the gel moldability.

**Table 6**

| | pH | Moldability | Skin touch | Adhesive performance | Pharmacological effect (early stage) |
|---|---|---|---|---|---|
| Example 1 | 4.8 | O | Δ | O | + |
| Example 2 | 3.8 | Δ | Δ | O | + |
| Comparative Example 3 | 4.2 | Δ | Δ | O | ± |
| Comparative Example 4 | 5.0 | O | O | Δ | (±) |
| Comparative Example 5 | 6.0 | O | O | Δ | (±) |

### <Experiment 3>

The crosslinking agents in the adhesive gel compositions of the present invention were compared with each other, and the effect of the addition of a polyhydric alcohol was evaluated. As shown in Table 7, using lidocaine and epinephrine as basic drugs and a polyfunctional epoxy compound or a metal compound as a crosslinking agent, the adhesive compositions of Example 3 and Comparative Example 6 were prepared. The adhesive composition of Example 4 in which a gelatin was further added was also prepared.

**Table 7**

| Adhesive composition | Crosslinking agent, Polyfunctional epoxy compound | Crosslinking agent, Metal compound |
|---|---|---|
| Without gelatin | Example 3 | - |
| With gelatin | Example 4 | Comparative Example 6 |

### (Example 3)

| | |
|---|---|
| Polyacrylic acid | 6 Parts by weight |
| Glycerin | 40 Parts by weight |
| Water | 45.6 Parts by weight |
| Lidocaine | 8 Parts by weight |
| Epinephrine . | 0.1 Part by weight |
| Sodium hydrogen sulfite | 0.3 Part by weight |

These components in the above formulation were mixed and stirred with heating at 50°C until a uniform mixture was obtained and epinephrine, an antioxidant and a crosslinking agent were added finally at 40°C to obtain an adhesive composition of the present invention. The composition was plastered onto a release-treated PET liner to have a thickness of 1 mm, and cut into a suitable form and subjected to a test.

### (Example 4)

| | |
|---|---|
| Polyacrylic acid | 6 Parts by weight |
| Glycerin | 40 Parts by weight |
| Ethylene glycol diglycidylether | 0.1 Part by weight |
| Gelatin | 5 Parts by weight |
| Water | 40.5 Parts by weight |
| Lidocaine | 8 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 6)

| | |
|---|---|
| Polyacrylic acid | 6 Parts by weight |
| Glycerin | 40 Parts by weight |
| Aluminum hydroxide | 1 Part by weight |
| Gelatin | 5 Parts by weight |
| Water | 39.6 Parts by weight |
| Lidocaine | 8 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

As shown in Table 8, the adhesive compositions of Examples 3 and 4 exhibit excellent characteristics in all of the gel moldability, the skin touch and the adhesive performance. On the other hand, in the adhesive composition of Comparative Example 6, moldability of the gel was poor, exudation was observed, and skin touch was also poor. In the adhesive composition of Comparative Example 6, epinephrine and aluminum were reacted, and a coloration of the gel during storage was observed. With regard to the pharmacological effect, each of Examples 3 and 4 exhibited a high local anesthetic effect, while Comparative Example 6 exhibited a tendency of a reduction. In addition, the composition of Example 4 containing a gelatin showed an advantage of being eawsy in handling upon manufacturing such as plastering and cutting.

**Table 8**

| Evaluation | pH | Moldability | Skin touch | Adhesive performance | Pharmaceutical effect (early stage) |
|---|---|---|---|---|---|
| Example 3 | 4.0 | O | Δ | O | + |
| Example 4 | 4.2 | O | O | O | + |
| Comparative Example 6 | 4.0 | × | × | Δ | ± |

### <Experiment 4>

The effect of the weight ratio of a local anesthetic and an acidic polymer on the moldability, the adhesive performance and the pharmacological effect of an adhesive gel composition of the present invention were evaluated. As shown in Table 9, 2 to 12 % of lidocaine and 0.1 % of epinephrine as basic drugs and 2 to 8 % of a polyacrylic acid as an acidic polymer were employed to prepare the adhesive compositions of Examples 5 to 10 and Comparative Examples 7 to 9.

**Table 9**

| Lidocaine (%) | Polyacrylic acid | | | |
|---|---|---|---|---|
| | 2% | 4% | 6% | 8% |
| 2% | - | - | - | Comparative Example 7 |
| 4% | - | Example 5 | Example 6 | Example 7 |
| 8% | Comparative Example 8 | Example 8 | Example 9 | Example 10 |
| 12% | - | - | Comparative Example 9 | - |

### (Example 5)

| | |
|---|---|
| Polyacrylic acid | 4 Parts by weight |
| Glycerin | 40 Parts by weight |
| Ethylene glycol diglycidylether | 0.1 Part by weight |
| Gelatin | 4.0 Parts by weight |
| Water | 47.5 Parts by weight |
| Lidocaine | 4 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Example 6)

| | |
|---|---|
| Polyacrylic acid | 6 Parts by weight |
| Glycerin | 40 Parts by weight |
| Ethylene glycol diglycidylether | 0.1 Part by weight |
| Gelatin | 4.0 Parts by weight |
| Water | 45.5 Parts by weight |
| Lidocaine | 4 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Example 7)

| | |
|---|---|
| Polyacrylic acid | 8 Parts by weight |
| Glycerin | 40 Parts by weight |
| Ethylene glycol diglycidylether | 0.1 Part by weight |
| Gelatin | 4.0 Parts by weight |
| Water | 43.5 Parts by weight |
| Lidocaine | 4 Parts by weight |
| Epinephrine | 0.1 Part by weight |
| Sodium hydrogen sulfite | 0.3 Part by weight |

These components in the above formulation were mixed and stirred with heating at 40°C until a uniform mixture was obtained,and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Example 8)

| | |
|---|---|
| Polyacrylic acid | 4 Parts by weight |
| Glycerin | 40 Parts by weight |
| Ethylene glycol diglycidylether | 0.1 Part by weight |
| Gelatin | 4.0 Parts by weight |
| Water | 47.5 Parts by weight |
| Lidocaine | 4 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Example 9)

| | |
|---|---|
| Polyacrylic acid | 6 Parts by weight |
| Glycerin | 40 Parts by weight |
| Ethylene glycol diglycidylether | 0.1 Part by weight |
| Gelatin | 4.0 Parts by weight |
| Water | 41.5 Parts by weight |
| Lidocaine | 8 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Example 10)

| | |
|---|---|
| Polyacrylic acid | 8 Parts by weight |
| Glycerin | 40 Parts by weight |
| Ethylene glycol diglycidylether | 0.1 Part by weight |
| Gelatin | 4.0 Parts by weight |
| Water | 39.5 Parts by weight |
| Lidocaine | 8 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 7)

| | |
|---|---|
| Polyacrylic acid | 8 Parts by weight |
| Glycerin | 40 Parts by weight |
| Ethylene glycol diglycidylether | 0.1 Part by weight |
| Gelatin | 4.0 Parts by weight |
| Water | 45.5 Parts by weight |
| Lidocaine | 2 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 8)

| | |
|---|---|
| Polyacrylic acid | 2 Parts by weight |
| Glycerin | 40 Parts by weight |
| Ethylene glycol diglycidylether | 0.1 Part by weight |
| Gelatin | 4.0 Parts by weight |
| Water | 45.5 Parts by weight |
| Lidocaine | 8 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 9)

| | |
|---|---|
| Polyacrylic acid | 6 Parts by weight |
| Glycerin | 40 Parts by weight |
| Ethylene glycol diglycidylether | 0.1 Part by weight |
| Gelatin | 4.0 Parts by weight |
| Water | 37.5 Parts by weight |
| Lidocaine | 12 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

As shown in Table 10, each of the adhesive compositions of Examples 5 to 10 exhibited excellent characteristics in all of the moldability, the adhesive performance and the pharmacological effect. On the contrary, the composition of Comparative Example 7 had a smaller amount of the polymer, and gave an adhesive composition whose moldability was insufficient. The weight ratio of an acidic polymer to a basic drug in Comparative Examples 8 and 9 failed to achieve a sufficient neutralization and gave an insufficient moldability. In particular, in the composition of Comparative Example 8, the dissolution of the basic agent was poor, and a crystal was observed in the gel. Such adhesive composition exhibited an extremely poor adhesive performance.

**Table 10**

| | Polyacrylic acid (%) | Basic drug (%) | Polyacrylic acid to Basic drug | Moldability | Adhesive performance | Pharmacological effect (early stage) |
|---|---|---|---|---|---|---|
| Example 5 | 4 | 4.1 | About 1:1 | O O | | + |
| Example 6 | 6 | 4.1 | About 3:2 | O | O | + |
| Example 7 | 8 | 4.1 | About 2:1 | O | O | + |
| Example 8 | 4 | 8.1 | About 1:2 | O O | | + |
| Example 9 | 6 | 8.1 | About 3:4 | O O | | + |
| Example 10 | 8 | 8.1 | About 1:1 | | O O | + |
| Comparative Example 7 | 8 | 2.1 | About 4:1 | Δ | Δ | ± |
| Comparative Example 8 | 2 | 8.1 | About 1:4 | × | × | - |
| Comparative Example 9 | 6 | 12.1 | About 1:2 | Δ | Δ | + |

### <Experiment 5>

The effect of the weight ratio of lidocaine to epinephrine on the moldability, the adhesive performance and the pharmacological effect of an adhesive gel composition of the present invention were evaluated. As shown in Table 11, 0 to 10 % of lidocaine and 0 to 0.5 % of epinephrine were employed to prepare the adhesive compositions of Examples 11 to 30 and Comparatives 10 to 19. The amount of an acidic polymer added in a composition was adjusted on the basis of the amount of a basic drug.

**Table 11**

| Epinephrine concentration (%) | Lidocaine concentration(%) | | | | |
|---|---|---|---|---|---|
| | 0 | 1 | 4 | 8 | 10 |
| 0 | Comparative Example 10 | Comparative Example 16 | Comparative Example 17 | Comparative Example 18 | Comparative Example 19 |
| 0.010 | Comparative Example 11 | Example 11 | Example 16 | Example 21 | Example 26 |
| 0.050 | Comparative Example 12 | Example 12 | Example 17 | Example 22 | Example 27 |
| 0.100 | Comparative Example 13 | Example 13 | Example 18 | Example 23 | Example 28 |
| 0.250 | Comparative Example 14 | Example 14 | Example 19 | Example 24 | Example 29 |
| 0.500 | Comparative Example 15 | Example 15 | Example 20 | Example 25 | Example 30 |

### (Examples 11 to 15)

| | |
|---|---|
| Polyacrylic acid | 4 Parts by weight |
| Glycerin | 40 Parts by weight |
| Ethylene glycol diglycidylether | 0.1 Part by weight |
| Gelatin | 4 Parts by weight |
| Water | 50.1 to 50.59 Parts by weight |
| Lidocaine | 1 Part by weight |
| Epinephrine | 0.010 to 0.500 Part by weight |
| Sodium hydrogen sulfite. | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Examples 16 to 20)

| | |
|---|---|
| Polyacrylic acid | 6 Parts by weight |
| Glycerin | 40 Parts by weight |
| Ethylene glycol diglycidylether | 0.1 Part by weight |
| Gelatin | 4 Parts by weight |
| Water | 45.1 to 45.59 Parts by weight |
| Lidocaine | 4 Parts by weight |
| Epinephrine | 0.010 to 0.500 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Examples 21 to 25)

| | |
|---|---|
| Polyacrylic acid | 6 Parts by weight |
| Glycerin | 40 Parts by weight |
| Ethylene glycol diglycidylether | 0.1 Part by weight |
| Gelatin | 4 Parts by weight |
| Water | 41.1 to 41.59 Parts by weight |
| Lidocaine | 8 Parts by weight |
| Epinephrine | 0.010 to 0.500 Part by weight |
| Sodium hydrogen sulfite | 0.3 Part by weight |

These components in the above formulation were mixed and stirred with heating at 50°C until a uniform mixture was obtained and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Example 26 to 30)

| | |
|---|---|
| Polyacrylic acid | 8 Parts by weight |
| Glycerin | 40 Parts by weight |
| Ethylene glycol diglycidylether | 0.1 Part by weight |
| Gelatin | 4 Parts by weight |
| Water | 37.1 to 37.59 Parts by weight |
| Lidocaine | 10 Parts by weight |
| Epinephrine | 0.010 to 0.500 Part by weight |
| Sodium hydrogen sulfite | 0.3 Part by weight |

The components in the above formulation were mixed and stirred with heating at 50°C until a uniform mixture was obtained and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Comparative 10 to 15)

| | |
|---|---|
| Polyacrylic acid | 4 Parts by weight |
| Glycerin | 40 Parts by weight |
| Ethylene glycol diglycidylether | 0.1 Part by weight |
| Gelatin | 4 Parts by weight |
| Water | 51.1 to 51.6 Parts by weight |
| Epinephrine | 0 to 0.500 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 16)

| | |
|---|---|
| Polyacrylic acid | 4 Parts by weight |
| Glycerin | 40 Parts by weight |
| Ethylene glycol diglycidylether | 0.1 Part by weight |
| Gelatin | 4 Parts by weight |
| Water | 50.9 Parts by weight |
| Lidocaine | 1 Part by weight |

These components in the above formulation were mixed and stirred with heating at 50°C until a uniform mixture was obtained, and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 17)

| | |
|---|---|
| Polyacrylic acid | 6 Parts by weight |
| Glycerin | 40 Parts by weight |
| Ethylene glycol diglycidylether | 0.1 Part by weight |
| Gelatin | 4 Parts by weight |
| Water | 45.9 Parts by weight |
| Lidocaine | 4 Parts by weight |

These components in the above formulation were mixed and stirred with heating at 50°C until a uniform mixture was obtained, and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 18)

| | |
|---|---|
| Polyacrylic acid | 6 Parts by weight |
| Glycerin | 40 Parts by weight |
| Ethylene glycol diglycidylether | 0.1 Part by weight |
| Gelatin | 4 Parts by weight |
| Water | 41.9 Parts by weight |
| Lidocaine | 8 Parts by weight |

These components in the above formulation were mixed and stirred with heating at 50°C until a uniform mixture was obtained, and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 19)

| | |
|---|---|
| Polyacrylic acid | 8 Parts by weight |
| Glycerin | 40 Parts by weight |
| Ethylene glycol diglycidylether | 0.1 Part by weight |
| Gelatin | 4 Parts by weight |
| Water | 37.9 Parts by weight |
| Lidocaine | 10 Parts by weight |

These components in the above formulation were mixed and stirred with heating at 50°C until a uniform mixture was obtained, and a crosslinking agent were added finally at 40°C to obtain a comparative adhesive composition. The composition was plastered onto a release-treated PET liner to have a thickness of 1 mm and cut into a suitable form and subjected to a test.

Using the adhesive compositions of Examples 11 to 30 and Comparative Examples 10 to 19, a comparative test shown in Table 12 was performed. Since the moldability, the adhesive performance and the neutralization of a polyacrylic acid were insufficient with lidocaine at the concentrations of 0 % (Comparative Examples 10 to 15) and 1 % (Comparative Examples 16 and Examples 11 to 15), the gels were poor in moldability. On the other hand, a composition containing lidocaine at a concentration of 4 % or higher gave a gel which had excellent moldability and adhesive performance when the amount of a polyacrylic acid was adjusted suitably.

The local anesthetic effect was not observed in Comparative Examples 11 to 15 which employed no lidocaine. The effect was observed at an early stage in Comparative Examples 16 to 19 which employed no epinephrine, but the effect was not sustained and disappeared within a short period. On the contrary, a significantly sustained effect was observed when epinephrine was added at a concentration of 0.01 % or higher. Thus, a ratio of lidocaine to epinephrine within the range of 1000:1 to 2:1 showed a sustained pharmacological effect.

**Table 12**

| | Lidocaine (LC, %) | Epinephrine (EPI, %) | LC:EPI | Moldability | Adhesive performance | Pharmacological effect (early stage) | Pharmacological effect (sustained effect) |
|---|---|---|---|---|---|---|---|
| Example 11 | 1 | 0.01 | 100:1 | Δ | Δ | + | Δ |
| Example 12 | 1 | 0.05 | 20:1 | Δ | Δ | + | Δ |
| Example 13 | 1 | 0.10 | 10:1 | Δ | Δ | + | Δ |
| Example 14 | 1 | 0.25 | 4:1 | Δ | Δ | + | Δ |
| Example 15 | 1 | 0.50 | 2:1 | Δ | Δ | + | Δ |
| Example 16 | 4 | 0.01 | 400:1 | O | O | ++ | O |
| Example 17 | 4 | 0.05 | 80:1 | O | O | ++ | O |
| Example 18 | 4 | 0.10 | 40:1 | O | O | ++ | O |
| Example 19 | 4 | 0.25 | 16:1 | O | O | ++ | O |
| Example 20 | 4 | 0.50 | 8:1 | O | O | ++ | O |
| Example 21 | 8 | 0.01 | 800:1 | O | O | ++ | O |
| Example 22 | 8 | 0.05 | 160:1 | O | O | ++ | O |
| Example 23 | 8 | 0.10 | 80:1 | O | O | ++ | O |
| Example 24 | 8 | 0.25 | 32:1 | O | O | ++ | O |
| Example 25 | 8 | 0.50 | 16:1 | O | O | ++ | O |
| Example 26 | 10 | 0.01 | 1000:1 | O | O | ++ | O |
| Example 27 | 10 | 0.05 | 200:1 | O | O | ++ | O |
| Example 28 | 10 | 0.10 | 100:1 | O | O | ++ | O |
| Example 29 | 10 | 0.25 | 40:1 | O | O | ++ | O |
| Example 30 | 10 | 0.50 | 32:1 | O | O | ++ | O |
| Comparative Example 10 | 0 | 0 | | × | Δ | - | × |
| Comparative Example 11 | 0 | 0.01 | | × | Δ | - | × |
| Comparative Example 12 | 0 | 0.05 | | × | Δ | - | × |
| Comparative Example 13 | 0 | 0.10 | | × | Δ | - | × |
| Comparative Example 14 | 0 | 0.25 | | × | Δ | - | × |
| Comparative Example 15 | 0 | 0.50 | | × | Δ | - | × |
| Comparative Example 16 | 1 | 0 | | Δ | Δ | ± | × |
| Comparative Example 17 | 4 | 0 | | O | O | + | × |
| Comparative Example 18 | 8 | 0 | | O | O | + | × |
| Comparative Example 19 | 10 | 0 | | O | O | + | × |

### <Experiment 6>

The effect of the current amount on the pharmacological effect of an adhesive gel composition of the present invention was evaluated. In the presence of 8 % lidocaine and 0.1 % epinephrine with the current of 0.1 mA/cm², Examples 31 to 34 as energization groups and Comparative Examples 20 to 23 as non-energization groups were compared during the application time period ranging from 5 to 30 minutes as shown in Table 13.

**Table 13**

| Application time | Energization group | Non-energization group |
|---|---|---|
| 5 minutes | Example 31 | Comparative Example 20 |
| 10 minutes | Example 32 | Comparative Example 21 |
| 15 minutes | Example 33 | Comparative Example 22 |
| 30 minutes | Example 34 | Comparative Example 23 |

### (Examples 31 to 34, Comparative Examples 20 to 23)

| | |
|---|---|
| Polyacrylic acid | 8 Parts by weight |
| Glycerin | 40 Parts by weight |
| Ethylene glycol diglycidylether | 0.1 Part by weight |
| Gelatin | 4 Parts by weight |
| Water | 39.5 Parts by weight |
| Lidocaine | 8 Parts by weight |
| Epinephrine | 0.1 Part by weight |
| Sodium hydrogen sulfite | 0.3 Part by weight |

These components in the above formulation were mixed and stirred with heating at 50°C until a uniform mixture was obtained, and epinephrine, an antioxidant and a crosslinking agent were added finally at 40°C to obtain a comparative adhesive composition. The composition was plastered onto a release-treated PET liner to have a thickness of 1 mm, and cut into a suitable form and subjected to a test.

Before initiating the experiment, the dorsal region of a guinea pig was clipped using a clipper and an electric shaver, and the skin surface was wiped thoroughly with a gauze soaked with a slightly warm water. The right or left side of the dorsal medium line was stimulated with a stimulating needle, and a site where a skin constriction response was surely identified was employed as an adhesive gel-application site, and the remaining clipped region was applied with a control electrode. A drug-containing adhesive gel (donor electrode, 6 cm²) was connected to a cathode, and a sodium chloride-containing electrode (reference electrode, 6 cm²) was connected to an anode, whereby initiating the energization. In the non-energization group, the application was performed similarly but no electricity was supplied. The energization was performed with a direct current of 0.1 mA/cm² for 5 to 30 minutes.

As evident from Table 14, no local anesthetic effect was observed in any of the non-energization groups of Comparative Examples 20 to 23. On the other hand, each energization group exhibited a local anesthetic effect after 5 minutes or longer of the application, and an almost stable sustained local anesthetic effect was observed with a current of about 0.5 to 1.5 mA·min/cm². The effect, however, was rather constant even with a current of 1.5 mA·min/cm² or higher.

**Table 14**

| Application time period | Pharmacological effect (early stage) | Pharmacological effect (sustained effect) |
|---|---|---|
| Example 31 | + | Δ |
| Example 32 | ++ | O |
| Example 33 | ++ | O |
| Example 34 | ++ | O |
| Comparative Example 20 | - | × |
| Comparative Example 21 | - | × |
| Comparative Example 22 | - | × |
| Comparative Example 23 | - | × |

### <Experiment 7>

The effect of the concentration of an antioxidant (sodium hydrogen sulfite) on the stability of a vasoconstrictor (epinephrine) in an adhesive gel composition of the present invention, was investigated.

### (Example 35)

| | |
|---|---|
| Polyacrylic acid | 6 Parts by weight |
| Polyethlene Glycol 400 | 40 Parts by weight |
| Ethylene glycol diglycidylether | 0.1 Part by weight |
| Gelatin | 4 Parts by weight |
| Water | 46.54 Parts by weight |
| Lidocaine | 3 Parts by weight |
| Epinephrine | 0.05 Part by weight |
| Sodium hydrogen sulfite | 0.3 Part by weight |
| Oxyquinoline sulfate | 0.01 Part by weight |

These components in the above formulation were mixed and stirred with heating at 50°C until a uniform mixture was obtained, and a crosslinking agent were added finally at 40°C to obtain a comparative adhesive composition. The composition was plastered onto a release-treated PET liner to have a thickness of 1 mm, and cut into a suitable form and subjected to a test.

### (Example 36)

| | |
|---|---|
| Polyacrylic acid | 6 Parts by weight |
| Polyethylene Glycol 400 | 40 Parts by weight |
| Ethylene glycol diglycidylether | 0.1 Part by weight |
| Gelatin | 4 Parts by weight |
| Water | 46.24 Parts by weight |
| Lidocaine | 3 Parts by weight |
| Epinephrine | 0.05 Part by weight |
| Sodium hydrogen sulfite | 0.6 Part by weight |
| Oxyquinoline sulfate | 0.01 Part by weight |

These components in the above formulation were mixed and stirred with heating at 50°C until a uniform mixture was obtained, and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 24)

| | |
|---|---|
| Polyacrylic acid | 6 Parts by weight |
| Polyethylene glycol 400 | 40 Parts by weight |
| Ethylene glycol diglycidylether | 0.1 Part by weight |
| Gelatin | 4 Parts by weight |
| Water | 46.85 Parts by weight |
| Lidocaine | 3 Parts by weight |
| Epinephrine | 0.05 Part by weight |

These components in the above formulation were mixed and stirred with heating at 50°C until a uniform mixture was obtained, and a crosslinking agent were added finally at 40°C to obtain a comparative adhesive composition. The composition was plastered onto a release-treated PET liner to have a thickness of 1 mm, and cut into a suitable form and subjected to a test.

### (Comparative Example 25)

| | |
|---|---|
| Polyacrylic acid | 6 Parts by weight |
| Polyethylene glycol 400 | 40 Parts by weight |
| Ethylene glycol diglycidylether | 0.1 Part by weight |
| Gelatin | 4 Parts by weight |
| Water | 46.34 Parts by weight |
| Lidocaine | 3 Parts by weight |
| Epinephrine | 0.05 Part by weight |
| Sodium hydrogen sulfite | 1.5 Parts by weight |
| Oxyquinoline sulfate | 0.01 Part by weight |

These components in the above formulation were mixed and stirred with heating at 50°C until a uniform mixture was obtained, and a crosslinking agent were added finally at 40°C to obtain a comparative adhesive composition. The composition was plastered onto a release-treated PET liner to have a thickness of 1 mm, and cut into a suitable form and subjected to a test.

Using the adhesive gels of Examples 35, 36 and Comparative Examples 24 and 25, the stabilities of lidocaine and epinephrine were investigated. Each adhesive gel was stored at 50°C for 4 weeks, and then determined for the lidocaine content and the epinephrine content. As a result, about 100 % recovery of lidocaine was observed in each of Examples and Comparative Examples, as shown in Table 15, exhibiting the stability in the adhesive gels. On the other hand, % recoveries of epinephrine were about 96 % in Example 35, about 84 % in Example 36, about 67 % in Comparative Example 24 and about 72 % in Comparative Example 25, showing the stabilizing effect which varied depending on the concentration of sodium hydrogen sulfite added, especially at a high concentration of which a reduction in the stability of epinephrine was noted.

**Table 15**

| | Lidocaine content (% based on initial content) | Epinephrine content (% based on initial content) |
|---|---|---|
| Example 35 | 102.1 ± 0.4 | 95.7 ± 0.5 |
| Example 35 | 101.2 ± 4.0 | 83.7 ± 2.0 |
| Comparative Example 24 | 101.2 ± 3.4 | 67.4 ± 1.2 |
| Comparative Example 25 | 100.9 ± 2.2 | 72.3 ± 0.9 |

A content is represented as mean ± standard deviation (n=3).

### Industrial Applicability

As described above, an adhesive gel composition according to the present invention is applied to a skin or a mucosa, and comprises an acidic polymer, a polyfunctional epoxy compound, water, a polyhydric alcohol and/or a gelatin as well as a basic drug, which exhibits excellent moldability and adhesive performance without a reduction in the drug delivery, which was a disadvantageous aspect of a conventional base. The adhesive gel composition according to the present invention and the device therefore, ensures a high quality of any of the aspects including manufacture, handling, skin irritation, stability over a time period, pharmacological effect, electrical characteristics and cost efficiency, thus being suitable to be used in an iontophoresis in a medical field.

## Claims

1. An adhesive gel composition for an iontophoresis comprising (a) basic drug(s), an acidic polymer, a polyfunctional epoxy compound, water, a polyhydric alcohol and/or a gelatin.

2. An adhesive gel composition for an iontophoresis according to Claim 1 wherein the weight ratio of the basic drug(s) to the acidic polymer is 10:1 to 1:10.

3. An adhesive gel composition for an iontophoresis according to Claim 1 or 2 wherein the acidic polymer is one or more selected from the group consisting of a polyacrylic acid, a methoxyethylene maleic anhydride copolymer, a methoxyethylene maleic acid copolymer, an isobutylene maleic anhydride copolymer, an isobutylene maleic acid copolymer, a carboxyvinyl polymer and carboxymethyl cellulose.

4. An adhesive gel composition for an iontophoresis according to any of Claims 1 to 3 wherein the basic drug(s) is(are) in a free form.

5. An adhesive gel composition for an iontophoresis according to Claim 4 wherein the basic drug is a local anesthetic agent.

6. An adhesive gel composition for an iontophoresis according to Claim 4 wherein the basic drugs are a local anesthetic agent and a vasoconstrictor.

7. An adhesive gel composition for an iontophoresis according to Claim 6 wherein the local anesthetic agent is lidocaine and the vasoconstrictor is epinephrine.

8. An adhesive gel composition for an iontophoresis according to Claim 7 wherein the weight ratio of lidocaine to epinephrine is 1000:1 to 2:1.

9. An adhesive gel composition for an iontophoresis according to Claim 7 comprising 1 to 20 % by weight of lidocaine and 0.001 to 0.5 % by weight of epinephrine.

10. An adhesive gel composition for an iontophoresis according to Claim 7 further comprising an antioxidant.

11. An adhesive gel composition for an iontophoresis according to Claim 10 wherein the antioxidant is one or more selected from the group consisting of sodium pyrosulfite, sodium hydrogen sulfite and oxyquinoline sulfate.

12. An adhesive gel composition for an iontophoresis according to Claim 10 or 11 comprising 0.001 to 1.0 % by weight of an antioxidant.

13. An iontophoresis device comprising a donor electrode, a reference electrode and a power supply connected electrically to each of the donor electrode and the reference electrode, wherein the current output from the source device is at least one of a direct current, a pulse direct current and a pulse depolarized direct current and wherein the current rate is 0.25 to 5.00 mA·min/cm².
